# EUROPEAN PATENT APPLICATION

(11) **EP 4 830 670 A1**
(43) Date of publication of application: **28.10.2026**
(21) Application number: 24912303.5
(22) Date of filing: 09.12.2024
(51) Int. Cl.: A61F 2/852

(54) **STENT**

(30) Priority: 25.12.2023 JP 2023218652
(71) Applicant: T.G. Medical Inc., Tokyo 103-0023 (JP)
(72) Inventor: MIKI, Kohei, Tokyo 103-0023 (JP)
(74) Representative: Appleyard Lees IP LLP
(86) International application number: PCT/JP2024/043334
(87) International publication number: WO 2025/142414

(57) **Abstract**

Provided is a stent that is unlikely to kink, can uniformly expand blood vessels, and can sufficiently ensure blood flow. A stent 1 includes: a first stent body 10 in which a plurality of first cells 12 surrounded by a plurality of first struts 11 are arrayed in a circumferential direction and a central axis direction; and a second stent body 20 in which a plurality of second cells 22 surrounded by a plurality of second struts 21 are arrayed in the circumferential direction and the central axis direction, and which is inserted into the first stent body 10. At least one of the first struts 11 and the second struts 21 has a portion in which the strut width w in a direction perpendicular to an extension direction of the first struts 11 or the second struts 21 is wider than the strut thickness t in a direction perpendicular to the central axis.

## Description

### TECHNICAL FIELD

The present disclosure relates to a stent.

### BACKGROUND ART

A catheter treatment in which a stent is deployed in a lesion area of a vascular lumen has been known.

In a conventional stent, a strut width is approximately equal to a strut thickness or the strut width is narrower than the strut thickness. This is because in the conventional stent, when the strut width is increased and the strut thickness is reduced, the stiffness of the stent is increased when the stent is narrowed, and for this reason, there is a probability that it is difficult to mount the stent on a small-diameter catheter and deliver the stent or that the stent buckles (kinks).

However, it is originally preferable that the stent have a large strut width and a large surface area of a surface contacting an inner wall of the blood vessel in order to uniformly expand the narrowed blood vessel. It is also originally preferable that the thickness of the strut be reduced to increase the cross-sectional area of the lumen when the stent is deployed in order to sufficiently ensure an antegrade blood flow. If the blood flow can be sufficiently ensured, the risk of thrombus attachment can be reduced, and therefore, it is originally preferable that the strut have a thin thickness also in this viewpoint. However, Patent Document 1 that discloses a stent does not disclose any design concept of a width and thickness of a strut.

### Citation List

### Patent Documents

Patent Document 1: Japanese Unexamined Patent Application, Publication No. 2020-78549

### DISCLOSURE OF THE INVENTION

### Problems to be Solved by the Invention

A problem to be solved by the present disclosure is to provide a stent that is unlikely to kink, can uniformly expand blood vessels, and can sufficiently ensure blood flow.

### Means for Solving the Problems

The present disclosure addresses the above-described problem by the following solution. Note that for the sake of easy understanding, the present disclosure will be described with reference numerals corresponding to an embodiment, but the disclosure is not limited thereto.

A first aspect of the present disclosure is directed to a stent (1) including: a first stent body (10) in which a plurality of first cells (12) including a plurality of first struts (11) are arrayed in a circumferential direction and a central axis direction; a second stent body (20) in which a plurality of second cells (22) including a plurality of second struts (21) are arrayed in the circumferential direction and the central axis direction and at least a region that expands a narrowed site of a living body lumen is inserted into the first stent body (10), in which at least one group of a first group of the first struts (11) and a second group of the second struts (21) has a portion in which a strut width w in a direction perpendicular to an extension direction of the first struts (11) or the second struts (21) is wider than a strut thickness t in a direction perpendicular to a central axis.

According to a second aspect of the present disclosure, the strut width w and the strut thickness t of the stent (1) of the first aspect satisfy 0.2 ≤ t/w.

According to a third aspect of the present disclosure, the strut width w of the stent (1) of the first aspect or second aspect satisfies 30 µm ≤ w ≤ 250 µm.

According to a fourth aspect of the present disclosure, the strut thickness t of the stent (1) of any one of the first to third aspects satisfies 20 µm ≤ t ≤ 150 µm.

According to a fifth aspect of the present disclosure, t1 > t2 is satisfied when the strut thickness t is defined as t1 for the first struts (11) or t2 for the second struts (21) in the stent (1) of any one of the first to fourth aspects.

According to a sixth aspect of the present disclosure, w1 > w2 is satisfied when the width w is defined as w1 for the first struts (11) or w2 for the second struts (21) in the stent (1) of any one of the first to fifth aspects.

According to a seventh aspect of the present disclosure, the stent (1) of any one of the first to sixth aspects is used for applications in which the stent (1) is temporarily placed in the living body lumen and subsequently retrieved from a body.

An eighth aspect of the present disclosure is directed to a method for expanding a living body organ with a lumen structure using a stent (1), the stent (1) including: a first stent body (10) in which a plurality of first cells (12) including a plurality of first struts (11) are arrayed in a circumferential direction and a central axis direction; and a second stent body (20) in which a plurality of second cells (22) including a plurality of second struts (21) are arrayed in the circumferential direction and the central axis direction and at least a region that expands a narrowed site of a living body lumen is inserted into the first stent body (10), in which at least one group of a first group of the first struts (11) and a second group of the second struts (21) has a portion in which a strut width w in a direction perpendicular to an extension direction of the first struts (11) or the second struts (21) is wider than a strut thickness t in a direction perpendicular to a central axis, the method including: delivering the stent (1) housed in a catheter (50) in a diameter-narrowed state together with the catheter (50), through the living body organ, to a position where the living body organ is to be expanded; retracting the catheter (50) to a proximal side and starting to expand the stent (1) from a distal side; completing expansion of the stent (1); and housing the expanded stent (1) in the catheter (50) again.

### Effects of the Invention

According to the present disclosure, it is possible to provide a stent which is unlikely to kink, uniformly expands blood vessels, and sufficiently ensures blood flow.

### BRIEF DESCRIPTION OF THE DRAWINGS

FIG. 1 is a side view of a stent 1 of a first embodiment;
FIG. 2 is a perspective view of the stent 1 shown in FIG. 1;
FIG. 3A is a virtual development view of a part of a first stent body 10 of the first embodiment;
FIG. 3B is a virtual development view of a part of a second stent body 20 of the first embodiment;
FIG. 3C is a virtual development view of a part of the stent 1 of the first embodiment;
FIG. 4A is a diagram for describing an outer diameter D1 of the first stent body 10 before assembly;
FIG. 4B is a diagram for describing an outer diameter D2 of the second stent body 20 before assembly;
FIG. 5 is a diagram showing a process of inserting the second stent body 20 into the first stent body 10;
FIG. 6 is a sectional view along an s1-s1 line of FIG. 1;
FIG. 7 is an enlarged sectional view showing a range B in FIG. 6;

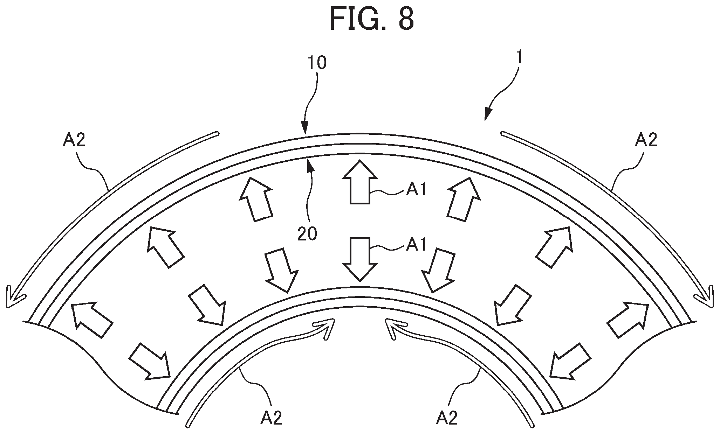

FIG. 8 is a diagram for describing an internal state when the stent 1 is bent;
FIG. 9 is a sectional view when a stent 1 of a second embodiment is cut at a position similar to that in FIG. 7 of the first embodiment;
FIG. 10 is a sectional view when a stent 1 of a third embodiment is cut at a position similar to that in FIG. 7 of the first embodiment;
FIG. 11 is a sectional view when a stent 1 of a fourth embodiment is cut at a position similar to that in FIG. 7 of the first embodiment;
FIG. 12 is a side view of a stent 1 of a fifth embodiment;
FIG. 13 is a perspective view of the stent 1 in which a distal side LD2 end portion is opened;
FIG. 14 is a side view of the stent 1 in which the distal side LD2 end portion is opened;
FIG. 15A is a schematic view showing steps of expanding a narrowed site in a blood vessel using the stent 1;
FIG. 15B is a schematic view showing steps of expanding a narrowed site in a blood vessel using the stent 1;
FIG. 15C is a schematic view showing steps of expanding a narrowed site in a blood vessel using the stent 1; and
FIG. 15D is a schematic view showing steps of expanding a narrowed site in a blood vessel using the stent 1.

### PREFERRED MODE FOR CARRYING OUT THE INVENTION

Hereinafter, embodiments of a stent will be described. Note that each of the drawings attached to the present specification shows a schematic view and the shape, scale, longitudinal-lateral dimensional ratio, etc. are changed or exaggerated as compared to actual shape, scale, longitudinal-lateral dimensional ratio, etc. Moreover, in the drawings, hatching showing the cross-section of members is omitted where appropriate.

In the present specification etc., terms specifying shapes, geometric conditions, and the degrees thereof, such as "perpendicular" and "direction", include not only exact meanings of these terms, but also ranges taken as being substantially perpendicular and being substantially in a direction. In description of the present specification etc., a side (a proximal side) close to a practitioner will be referred to as a side LD1 and a side (a distal side) distant from the practitioner will be referred to as a side LD2 in an axial direction (a longitudinal direction) LD. And a direction perpendicular to the axial direction LD will be referred to as a radial direction RD. Moreover, in description of the present specification etc., a direction in which cells are arrayed will be referred to as a circumferential direction OD. The circumferential direction may be not only the radial direction RD, but also a direction inclined with respect to the radial direction RD.

### (First Embodiment)

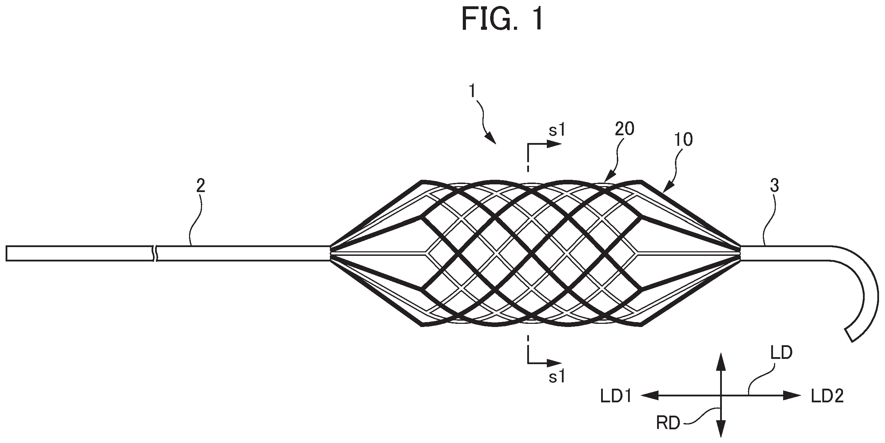

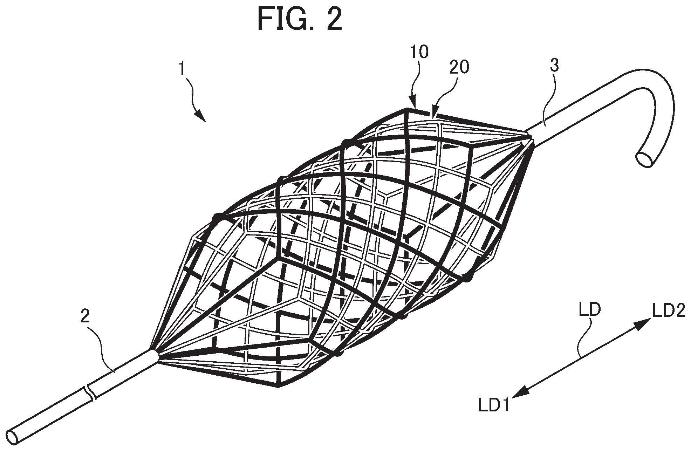

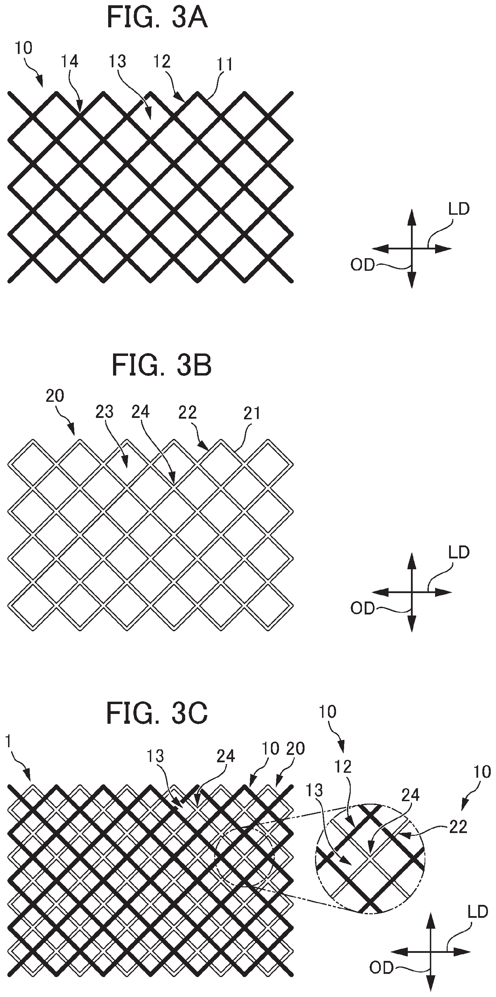

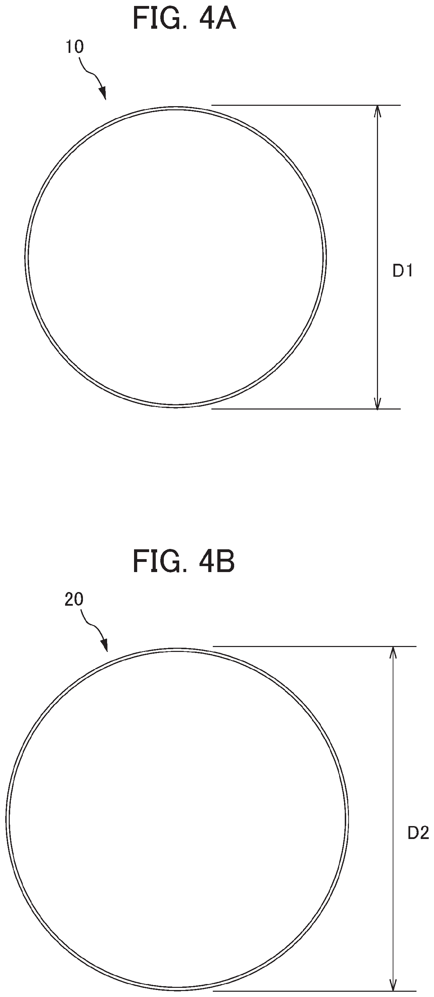

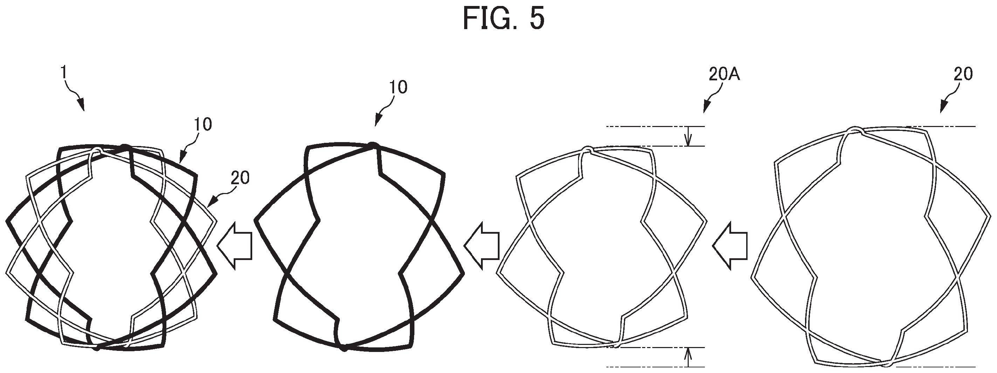

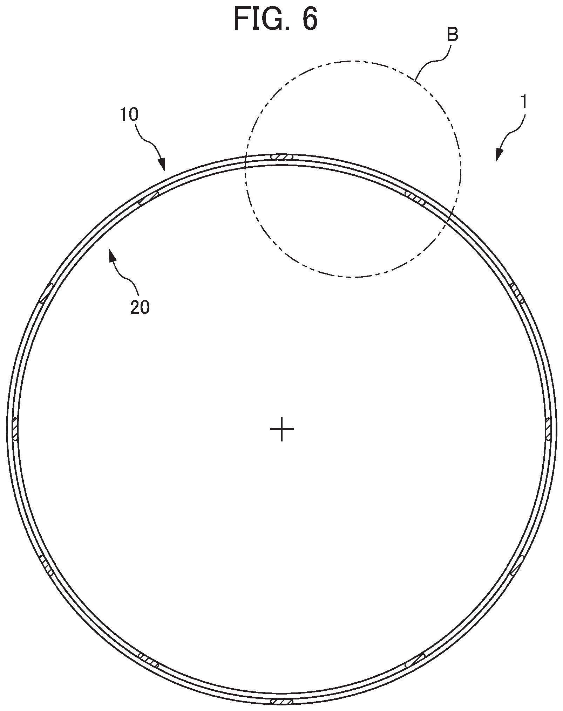

FIG. 1 is a side view of a stent 1 of a first embodiment. FIG. 2 is a perspective view of the stent 1 shown in FIG. 1. FIG. 3A is a virtual development view of a part of a first stent body 10 of the first embodiment. FIG. 3B is a virtual development view of a part of a second stent body 20 of the first embodiment. FIG. 3C is a virtual development view of a part of the stent 1 of the first embodiment. FIG. 4A is a diagram for describing an outer diameter D1 of the first stent body 10 before assembly. FIG. 4B is a diagram for describing an outer diameter D2 of the second stent body 20 before assembly. FIG. 5 is a diagram showing a process of inserting the second stent body 20 into the first stent body 10. FIG. 6 is a sectional view along an s1-s1 line of FIG. 1.

For the sake of easy distinguishing of the first stent body 10 and the second stent body 20 in the drawings, a strut of the first stent body 10 is indicated by black, and a strut of the second stent body 20 is indicated by white. Moreover, in the present specification etc., the "cell" indicates a portion surrounded by struts forming a stent. "Cells" may be identical to each other or different from each other in shape and size. The "strut" indicates a portion made of wire-like material. In the present specification etc., a cell opening will be referred to as an "opening portion", and a portion where struts of adjacent cells are connected to or overlap with each other will also be referred to as an "overlapping portion". Of the overlapping portion, a point at which struts cross each other will also be referred to as an "intersecting point". The overlapping portion may have a certain region (area). The overlapping portion may include a single or a plurality of intersecting points.

The stent 1 of the first embodiment may be used, for example, as follows: the stent 1 is sheathed (housed) in a catheter (not shown), pushed out of the catheter and expanded in a vascular lumen, such that the stent 1 expands a narrowed or occluded blood vessel. The stent 1 is used for an application to be retrieved from the body after having been temporarily placed in a blood vessel. Alternatively, the stent 1 may be used while remained in the blood vessel without being retrieved from the body. When the stent 1 is used for such an application, it may be preferable that a push wire 2 and the stent 1 are configured to be separable from each other through the operation by a practitioner.

As shown in FIGS. 1 and 2, the stent 1 is in a substantially cylindrical shape in a diameter-expanded state. The stent 1 is in an elongated cylindrical shape in a diameter-narrowed state. The push wire 2 is connected to a proximal side LD1 end portion of the stent 1, and a distal end shaft 3 is connected to a distal side LD2 end portion thereof. A method for connecting the proximal side end portion of the stent 1 and the push wire 2 to each other is not particularly limited as long as the connection method is used for general medical equipment. Examples of the method may include welding, UV bonding, and silver solder infiltration.

The practitioner pushes or pulls the push wire 2 via an operation unit (not shown) outside the body that is coupled to the proximal side LD1 of the push wire 2, thereby moving the stent 1 back and forth in the catheter or the blood vessel. The distal end shaft 3 is a mark for checking the distal side LD2 position of the stent 1 on an X-ray image, and at least a part of the distal end shaft 3 is made of radiopaque material.

The stent 1 includes the first stent body 10 and the second stent body 20, each of which is a substantially cylindrical structure. The stent 1 is a stent having such a double-layer structure in which the second stent body 20 is inserted into the first stent body 10 in at least a region that expands a narrowed site of a living body lumen. Here, the region that expands a narrowed site of a living body lumen refers to a region substantially contributing to expansion of the narrowed site during use. This region corresponds to a region expanding in a substantially cylindrical shape in a diameter-expanded state shown in FIGS. 1 and 2 in the stent 1 of the present embodiment. Moreover, the above-described "at least" means that in the above-described region that expands a narrowed site, the second stent body 20 is required to be inserted into the first stent body 10. Note that the region in which the second stent body 20 is inserted into the first stent body 10 is not limited to the above-described region that expands a narrowed site (in the example of the present embodiment, a region expanded in a substantially cylindrical shape). In the present embodiment, the second stent body 20 is inserted into the first stent body 10 immediately before the position where the first stent body 10 and the second stent body 20 are coupled to the push wire 2 and the distal end shaft 3.

The first stent body 10 and the second stent body 20 are not coupled to each other in the radial direction. Specifically, the first stent body 10 and the second stent body 20 are indirectly coupled to each other via the push wire 2 or the distal end shaft 3, but are not coupled to each other between the push wire 2 and the distal end shaft 3. Thus, the first stent body 10 and the second stent body 20 are independently and flexibly deformable.

Note that the first stent body 10 and the second stent body 20 are directly coupled to each other at a proximal end and one of the first stent body 10 and the second stent body 20 is coupled to the push wire 2. However, it may be configured such that the other of the first stent body 10 and the second stent body 20 is not coupled to the push wire 2. In other words, it is only required that the first stent body 10, the second stent body 20, and the push wire 2 are integrally connected to each other. That is, the proximal side end portion of the first stent body 10 and the proximal side end portion of the second stent body 20 are connected to the push wire 2 in an inseparable manner, and therefore, the second stent body 20 is restricted from moving in the axial direction relative to the first stent body 10. Similarly, the first stent body 10 and the second stent body 20 are directly coupled to each other at a distal end and one of the first stent body 10 and the second stent body 20 is coupled to the distal end shaft 3. However, it may be configured such that the other of the first stent body 10 and the second stent body 20 is not coupled to the distal end shaft 3. In other words, it is only required that the first stent body 10, the second stent body 20, and the distal end shaft 3 are integrally connected to each other. Note that at the proximal end or the distal end, the first stent body 10 and the second stent body 20 are connected to each other, but in the other portions, the first stent body 10 and the second stent body 20 are not coupled (connected) to each other in the radial direction. Also in a diameter-expanded state, the first stent body 10 and the second stent body 20 just closely contact to each other in the radial direction but are not coupled (connected) to each other in the radial direction. Thus, the first stent body 10 and the second stent body 20 are independently deformable, but do not restrict deformation of each other.

The proximal side LD1 of the first stent body 10, the proximal side LD1 of the second stent body 20, and the distal side LD2 of the push wire 2 are directly or indirectly connected to each other. Here, "directly connected" refers to, for example, a form in which the proximal side LD1 of the first stent body 10 and the distal side LD2 of the push wire 2 are connected to each other and the proximal side LD1 of the second stent body 20 and the distal side LD2 of the push wire 2 are connected to each other. In this case, a position where the proximal side LD1 of the first stent body 10 is connected to the push wire 2 may be the same as or different from a position where the proximal side LD1 of the second stent body 20 is connected to the push wire 2. On the other hand, "indirectly connected" refers to, for example, a form in which the proximal side LD1 of the first stent body 10 and the distal side LD2 of the push wire 2 are connected to each other and the proximal side LD1 of the first stent body 10 and the proximal side LD1 of the second stent body 20 are connected to each other (or a connection form in which the two stent bodies are connected in an inverse relationship). In this case, the proximal side LD1 of the second stent body 20 is indirectly connected to the push wire 2 via the proximal side LD1 of the first stent body 10. Alternatively, there may be another form in which the proximal side LD1 of the first stent body 10 and the proximal side LD1 of the second stent body 20 are connected to each other via a pipe-like sleeve (not shown) inserted over the distal side LD2 of the push wire 2. In this case, the proximal side LD1 of the first stent body 10 and the proximal side LD1 of the second stent body 20 are indirectly connected to the distal side LD2 of the push wire 2 via the sleeve. As described above, in the present specification, "indirectly connected" includes a form in which one of the proximal side LD1 of the first stent body 10 and the proximal side LD1 of the second stent body 20 is directly connected to the push wire 2, and the other stent body, which is not connected directly to the push wire 2, is indirectly connected to the push wire 2 via the stent body directly connected to the push wire 2. Examples of a method for connecting the proximal side end portion of each stent body and the push wire 2 to each other may include welding, UV bonding, and silver solder infiltration, but is not particularly limited as long as the connection method is used for general medical equipment.

As described later, the stent 1 of the first embodiment is produced in such a manner that the second stent body 20, which has a greater outer diameter than that of the first stent body 10 in a natural state, is inserted into the first stent body 10 in a diameter-narrowed state. Thus, the second stent body 20 constantly presses the first stent body 10 outward in the radial direction RD, so that the first stent body 10 and the second stent body 20 is closely in contact with each other.

As shown in FIG. 1, the proximal side LD1 end portions of the first stent body 10 and the second stent body 20, which are tapered and narrowed, are connected to the push wire 2. Similarly, the distal side LD2 end portions of the first stent body 10 and the second stent body 20, which are tapered and narrowed, are connected to the distal end shaft 3. Thus, the second stent body 20 is restricted from moving in the axial direction relative to the first stent body 10.

In the first stent body 10, as shown in FIG. 3A, a plurality of first cells 12 surrounded by first struts 11 are arrayed in a circumferential direction OD. In the first stent body 10, the plurality of first cells 12 are arrayed in the axis direction LD. That is, the first stent body 10 has such a pattern that the plurality of first cells 12 are arrayed in the circumferential direction OD and the axial direction LD. An adjacent pair of the first cells 12 is coupled to each other at an intersecting point 14.

In the second stent body 20, as shown in FIG. 3B, a plurality of inner cells (second cells) 22 surrounded by second struts 21 are arrayed in the circumferential direction OD. The plurality of second cells 22 are arrayed in the axis direction LD. That is, the second stent body 20 has such a pattern that the plurality of second cells 22 are arrayed in the circumferential direction OD and the axial direction LD. An adjacent pair of the second cells 22 is coupled to each other at an intersecting point 24.

As shown in FIGS. 3A and 3B, in the stent 1 of the first embodiment, a first cell 12 and a second cell 22 have the same size, shape, and arrangement, as one example. That is, in the first embodiment, the patterns shown in FIG. 3A and FIG. 3B are substantially the same. Note that the patterns of the first stent body 10 and the second stent body 20 may be different from each other.

As shown in FIG. 3C, the first stent body 10 and the second stent body 20 are arranged to overlap with each other in a natural state such that the intersecting point 24 between the adjacent second cells 22 (of the second stent body 20) is arranged in an opening portion 13 of the first cell 12 (of the first stent body 10). Specifically, one intersecting point 24 between the adjacent second cells 22 overlaps with one opening portion 13 of the first cell 12. Thus, the density of the struts can be increased over the entire stent, and the surface area of the stent 1 can be increased. In the stent 1 of the first embodiment, the percentage of a non-opening portion per area where the first stent body 10 and the second stent body 20 overlap with each other may be 7 to 45%.

In the first embodiment, a relationship between an outer diameter D1 of the first stent body 10 before assembly and an outer diameter D2 of the second stent body 20 before assembly is set to D1 < D2. Thus, as shown in FIG. 5, a second stent body 20A obtained by narrowing the second stent body 20 is inserted into the first stent body 10. As shown in FIG. 6, the second stent body 20 closely contacts the inside of the first stent body 10 in the circumferential direction OD due to expansive force of the second stent body 20 itself.

Thus, in the axial direction LD (see FIG. 1) of the stent 1, it is less likely that the positions of the first stent body 10 and the second stent body 20 are misaligned relative to each other. Note that for the sake of easy understanding, FIG. 5 shows only an annular circumferential cell line of each stent body.

In the first embodiment, the second stent body 20 inserted into the first stent body 10 in the diameter-narrowed state constantly presses the first stent body 10 outward in the radial direction RD. Consequently, even if the first stent body 10 and the second stent body 20 are not coupled to each other in the radial direction, the first stent body 10 and the second stent body 20 can closely contact each other. Moreover, since the first stent body 10 and the second stent body 20 are not coupled to each other in the radial direction in the stent 1, the first stent body 10 and the second stent body 20 are independently and flexibly deformable. Further, the stent 1 has the total expansive force created by the first stent body 10 and the second stent body 20. Thus, the stent 1 can have a greater ratio of the expansive force to the surface area than that of a stent having a single-layer structure.

The stent 1 (the first stent body 10, the second stent body 20) may be made of a material having a high biological compatibility. The stent 1 is preferably made of, but is not particularly limited to, a material having superelastic properties, such as nickel titanium (Ni-Ti) alloy. The first stent body 10 and the second stent body 20 may be produced, for example, in such a manner that substantially cylindrical tubes made of the above-described material are machined with laser.

The stent 1 may be loaded with a medical agent such as a physiologically active substance (a medical agent for inhibiting intima thickening, etc.), and specifically, may carry the medical agent so as to dissolve out the medical agent in the lumen structure.

For example, the first stent body 10 and the second stent body 20 are produced by machining tubes having diameters of about 0.4 to 3 mm with laser to form mesh patterns, and then, stretching the mesh patterns in the radial direction to expand to desired diameters. As described above, the second stent body 20 is inserted into the first stent body 10, and subsequently, the stent 1 having the double-layer structure can be produced. The stent 1 having the double-layer structure is sheathed in the catheter (not shown) while being narrowed in the radial direction from the natural state. The stent 1, which is made of, e.g., an elastic material such as superelastic alloy or shape-memory alloy, expands in diameter due to the self-expanding force when the stent 1 sheathed in the catheter is pushed out.

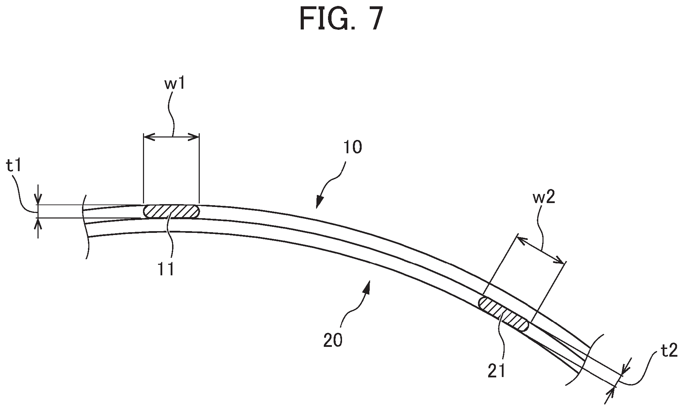

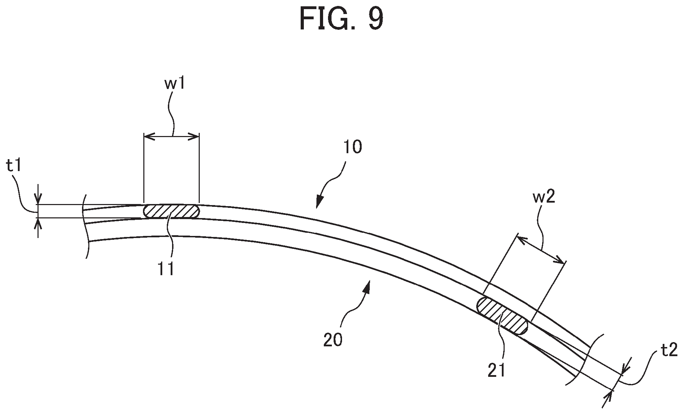

FIG. 7 is an enlarged sectional view showing a range B in FIG. 6. Note that in FIG. 7, sectional shapes of the first strut 11 and the second strut 21 are shown as sectional shapes in directions perpendicular to extension directions of the first strut 11 and the second strut 21 (the same applies to FIGS. 9, 10, and 11). As shown in FIG. 7, in both of the first strut 11 and the second strut 21, a strut width w (w1, w2) in a direction perpendicular to an extension direction of the first struts 11 or the second struts 21 is wider than a strut thickness t (t1, t2) in a direction perpendicular to the central axis of the stent 1. That is, the first strut 11 has a relationship of t1 < w1, and the second strut 21 has a relationship of t2 < w2. In the present embodiment, t1 = t2 = 60 µm, and w1 = w2 = 120 µm. It may be preferable that the strut thickness t (t1, t2) satisfy a relationship of 20 µm ≤ t ≤ 150 µm. This is because when the strut thickness t is smaller than 20 µm, the expansive force reduces, and for this reason, there is a probability that a narrowed lesion area may not be sufficiently expanded, and when the strut thickness t is larger than 150 µm, the expansive force increases, and for this reason, there is a probability that the blood vessel may excessively expand to cause blood vessel damage. It may be preferable that the strut width w (w1, w2) satisfy a relationship of 30 µm ≤ w ≤ 250 µm. This is because when the strut width w is smaller than 30 µm, a surface area of a portion contacting a lesion area reduces, and for this reason, there may be a probability that a force applied to a strut unit surface area increases and a strong force is locally applied to cause blood vessel damage. When, on the other hand, the strut width w is larger than 250 µm, the struts collide with each other when the stent is narrowed, and for this reason, there may be a probability that the stiffness of the stent increases in the catheter, making it difficult to deliver the stent to the lesion. Note that a range satisfying the relationship of 20 µm ≤ t ≤ 150 µm and a range satisfying the relationship of 30 µm ≤ w ≤ 250 µm may be applied to the entirety or a part of the range in which the second stent body 20 is inserted into the first stent body 10. It may be preferable to set the part of the range described above to be a region that expands a narrowed site of a living body lumen (in the example of the present embodiment, a region expanding in a substantially cylindrical shape). Alternatively, it may be possible to set the part of the range to be a narrower part of the above-mentioned region.

In the present embodiment, t < w (t1 < w1, t2 < w2) is satisfied, so that the surface area of the surface contacting the inner wall of the blood vessel can be significantly increased. Accordingly, it is possible to increase the uniformity in expanding the blood vessel. A large surface area reduces a pressure pressing the inner wall of the blood vessel, so that the risk of blood vessel damage can be reduced. When the strut width w is increased in a conventional manner in which t and w have a substantially equal dimension or a sectional shape in a direction perpendicular to an extension direction of the strut is a substantially square shape, the strut thickness t also increases, resulting in a reduction in the sectional area of the lumen when the stent is deployed. However, when the relationship of t < w is satisfied as in the present embodiment, the strut thickness t can be reduced, so that the sectional area of the lumen increases when the stent is deployed. Accordingly, it is possible to sufficiently ensure an antegrade blood flow and reduce the risk of thrombus attachment. It may be preferable to set the relationship between the strut thickness t (t1, t2) and the strut width w (w1, w2) to satisfy the relationship of 0.2 ≤ t/w. This is because when t/w < 0.2, the above-described various advantageous effects may not be sufficiently obtained. Note that a range satisfying the relationship of 0.2 ≤ t/w may be applied to the entirety or a part of the range in which the second stent body 20 is inserted into the first stent body 10. It may be preferable to set the part of the range described above to be a region in which a narrowed site of a living body lumen is expanded (in the example of the present embodiment, a region expanding in a substantially cylindrical shape). Alternatively, it may be possible to set the part of the range to be a narrower part of the above-mentioned region.

An effect in a case where the stent 1 of the first embodiment is removed after having been temporarily placed in a bent blood vessel will be described herein.

In the stent 1 of the present embodiment, the relationship of t < w (t1 < w1, t2 < w2) is satisfied as described above, so that the blood vessel can be expanded more uniformly, the risk of blood vessel damage can be reduced, the sectional area of the lumen when the stent is deployed can be increased, an antegrade blood flow can be sufficiently ensured, and the risk of thrombus attachment can be reduced. In a conventional stent having a single-layer structure, when the strut width w is increased and the strut thickness t is reduced, the stiffness of the stent increases when the stent is narrowed Accordingly, there has been a possibility that mounting or delivering the stent to a small-diameter catheter would be difficult and buckling (kinking) of the stent could occur. However, in the present embodiment, the stent having a double-layer structure is configured to satisfy t < w (t1 < w1, t2 < w2). The stent having a double-layer structure is divided into two layers even when the strut width w is widened and has a smaller density in the circumferential direction than that of a one-layer stent, so that the stent can be sheathed in the small-diameter catheter and an increase in the stiffness of the stent when the stent is narrowed can be reduced. For example, the stent 1 of the present embodiment can be sheathed in a small-diameter catheter having an inner diameter of 0.4 to 0.55 mm. The stent having a double-layer structure has a configuration in which the second stent body 20 constantly presses the first stent body 10 from inside as described above, so that the stent can be deployed without kinking during bending, even when being configured to satisfy t < w (t1 < w1, t2< w2).

The stent 1 of the first embodiment is deployed in a lesion area of a vascular lumen, and in this manner, the vascular lumen can be expanded, and the patency of the lesion area can be ensured. The stent 1 is removed (retrieved) from the vascular lumen in a predetermined period of time so that occurrence of restenosis or reocclusion or occurrence of a complication such as a thrombosis caused by an indwelling stent can be reduced. Since the stent 1 of the first embodiment has excellent shape followability, the stent 1 possesses high vascular preservation. Note that the stent 1 of the first embodiment can be used not only for treatment of a blood vessel but also for treatment of lumen structures such as the esophagus and large intestine.

The stent 1 of the first embodiment can also be used for treatment of a cerebrovascular spasm. Note that stents of other embodiments to be described later also produce advantageous effects similar to those of the stent 1 of the first embodiment.

### (Second Embodiment)

FIG. 9 is a sectional view when a stent 1 of a second embodiment is cut at a position similar to that in FIG. 7 of the first embodiment. The stent 1 of the second embodiment is similar to the stent 1 of the first embodiment except that the thickness t and width w of a strut are changed from those of the stent 1 of the first embodiment. Thus, the same reference numerals are used as elements having functions similar to those of the above-described first embodiment, and overlapping description thereof will be omitted as necessary.

The second embodiment is similar to the first embodiment in that the width w1 of a first strut is equal to the width w2 of a second strut, but satisfies the relationship of "the first strut thickness t1 < the second strut thickness t2". That is, the thickness t of a second stent body 20 arranged inside is thicker than the thickness t of a first stent arranged outside. Thus, in the stent 1 of the second embodiment, it is possible to increase a pressing force (a radial force) in which the second stent body 20 presses a first stent body 10 outward. In the present embodiment, t1 is set to 40 µm and t2 is set to 80 µm, but these numerical values may be changed as necessary. Note that in order to increase the radial force more effectively, it may be preferable to satisfy 1.2 < t2/t1. In the second embodiment, only t2 is made thicker than that of the first embodiment and t1 is set to a similar thickness to that of the first embodiment. Accordingly, the radial force can be increased, the kink resistance can be improved, so that the deployment property can be enhanced while a decrease in sectional area of the lumen is controlled when the stent is deployed, and. More specifically, the sectional area of the lumen increases when the thickness of the stent is reduced. However, it may be possible that disadvantages such as a reduction in kink resistance and expansive force would occur. However, in the stent 1 of the present embodiment, the second stent body 20 presses the first stent body 10 from inside, and in this manner, the kink resistance is enhanced. Thus, by reducing the thickness of only the first stent body 10, instead of reducing the thicknesses of both the first stent body 10 and the second stent body 20, all of the sectional area of the lumen, the kink resistance, and the expansive force can be appropriate.

### (Third Embodiment)

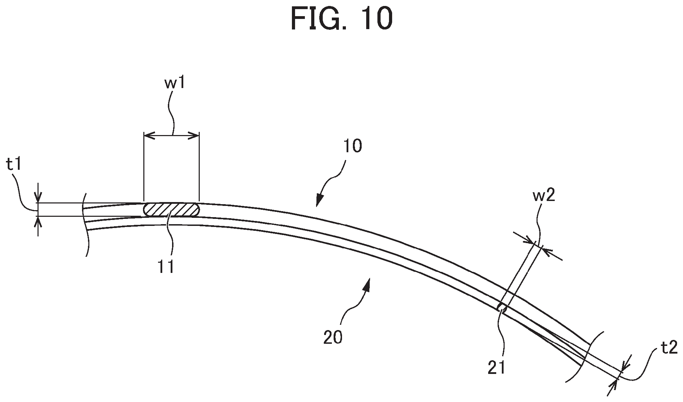

FIG. 10 is a sectional view when a stent 1 of a third embodiment is cut at a position similar to that in FIG. 7 of the first embodiment. The stent 1 of the third embodiment is similar to the stent 1 of the first embodiment except that the thickness t and width w of a strut are changed from those of the stent 1 of the first embodiment. Thus, the same reference numerals are used as elements having functions similar to those of the above-described first embodiment, and overlapping description thereof will be omitted as necessary.

In the third embodiment, a first strut 11 satisfies the relationship of t1 < w1 as in the first embodiment, but a second strut 21 is configured to satisfy t2 = w2. In the third embodiment, to satisfy t1 > t2 and w1 > w2, the thickness and width of the second strut 21 are configured to be smaller than those of the first strut 11. Such a configuration makes it easier to narrow a second stent body 20, which is narrowed to be smaller than a first stent body 10 in a narrowed state. Accordingly, it is possible to increase the sheathing property when the stent is sheathed in a catheter (not shown). In the third embodiment, the stent is configured to satisfy the relationships of both of t1 > t2 and w1 > w2. Alternatively, for example, it may be configured to satisfy the relationships of t1 > t2 and w1 = w2 or to satisfy the relationships of t1 = t2 and w1 > w2. Note that a range satisfying the relationships of t1 > t2 and w1 > w2 may be applied to the entirety or a part of the range in which the second stent body 20 is inserted into the first stent body 10. It may be preferable to set the part of the range described above to be a region that expands a narrowed site of a living body lumen (in the example of the present embodiment, a region expanding in a substantially cylindrical shape). Alternatively, it may be possible to set the part of the range to be a narrower part of the above-mentioned region.

### (Fourth Embodiment)

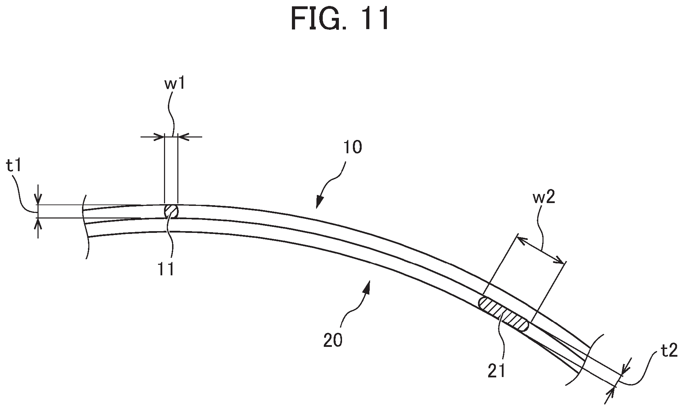

FIG. 11 is a sectional view when a stent 1 of a fourth embodiment is cut at a position similar to that in FIG. 7 of the first embodiment. The stent 1 of the fourth embodiment is similar to the stent 1 of the first embodiment except that the thickness t and width w of a strut are changed from those of the stent 1 of the first embodiment. Thus, the same reference numerals are used as elements having functions similar to those of the above-described first embodiment, and overlapping description thereof will be omitted as necessary.

In the fourth embodiment, a second strut 21 satisfies the relationship of t2 < w2 as in the first embodiment, but a first strut 11 is configured to satisfy t1 = w1. With such a configuration, an area of the second strut 21 contacting the first strut 11 can be increased, the second strut 21 can press the first strut 11 more stably, and the expansibility can be improved.

### (Fifth Embodiment)

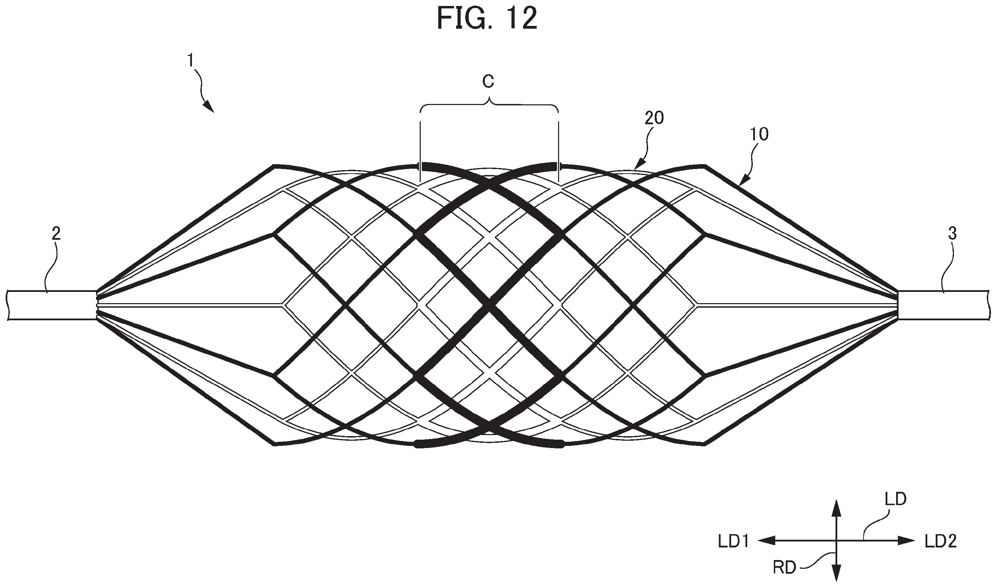

FIG. 12 is a side view of a stent 1 of a fifth embodiment. A stent 1 of the fifth embodiment is similar to the stent 1 of the first embodiment except that the thickness t and width w of a strut are partially changed, except for a range C, from those of the stent 1 of the first embodiment. Thus, the same reference numerals are used as elements having functions similar to those of the above-described first embodiment, and overlapping description thereof will be omitted as necessary.

In the fifth embodiment, the range C shown in FIG. 12 is configured to satisfy t < w (t1 < w1, t2 < w2) as in the first embodiment, but the ranges except for the range C are configured to satisfy t = w (t1 = w1, t2 = w2) in a conventional manner. With such a configuration, the width and thickness of only the range C which is a portion corresponding to a narrowed lesion area are increased. Accordingly, the effect of expanding the narrowed lesion area can be enhanced. On the other hand, since portions other than the range C contact normal blood vessels, the expansion force can be reduced by reducing the width and thickness. Accordingly, the safety can be enhanced without applying an excessive force to the normal blood vessels. Since the stiffness of the stent is also reduced when the stent is narrowed, the diameter-narrowing performance can be also improved.

### (Usage Method)

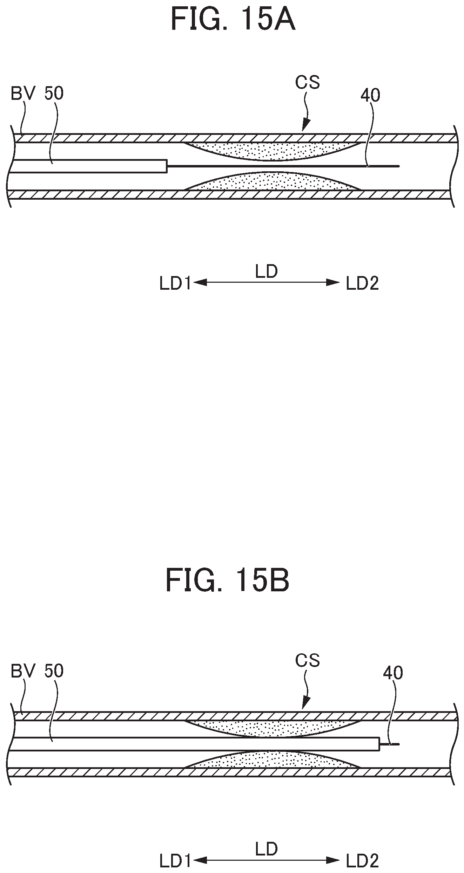

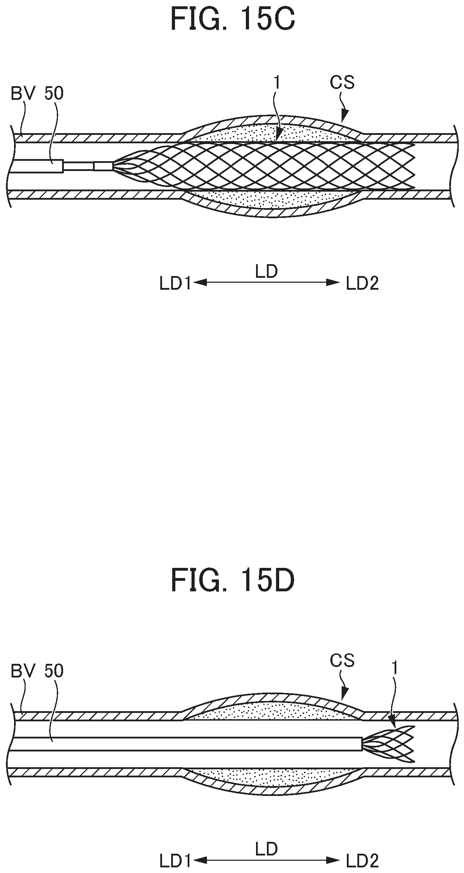

Next, one example of a usage method of the stent 1 of each embodiment will be described. FIGS. 15A to 15D each are a schematic view showing steps of expanding a narrowed site in a blood vessel using the stent 1. In FIGS. 15C and 15D, the shape of the stent 1 is shown in a simplified manner. Here, an operation of expanding a narrowed site CS formed in a blood vessel BV to ensure blood flow will be described. Note that the following operation is performed while grasping a position of the stent or the like using a visual marker (a radiopaque marker).

First, as shown in FIG. 15A, a guide wire 40 is passed through the narrowed site CS. The catheter 50 is inserted over the guide wire 40. Next, as shown in FIG. 15B, a distal end of the catheter 50 inserted over the guide wire 40 is inserted into the narrowed site CS along the passed guide wire 40, and is passed to reach a distal LD2 side of the narrowed site CS. Next, although not shown in the figure, the guide wire 40 is drawn to retrieve from the body through the catheter 50. Then, the stent 1 is inserted from a proximal LD1 side of the catheter 50. The stent 1 is inserted into the catheter 50 in a diameter-narrowed state.

Next, as shown in FIG. 15C, the stent 1 is deployed from the distal end of the catheter 50 in the distal LD2 side of the narrowed site CS. The distal end of the catheter 50 is located at the distal LD2 side of the narrowed site CS and the catheter 50 is drawn to the proximal LD1 side, so that the stent 1 can be deployed from the distal end of the catheter 50. When the stent 1 is deployed from the distal end of the catheter 50, the stent 1 is expanded by a self-expanding force, so that the narrowed site CS is expanded from the inside. The narrowed site CS is expanded from the inside by the expanded stent 1, so that the blood flow in the blood vessel BV can be ensured.

After it is checked that the blood flow in the blood vessel BV has been ensured, for example, observation of about 5 to 60 minutes is performed. After the observation, as shown in FIG. 15D, the distal end of the catheter 50 is advanced to the distal LD2 side of the stent 1, and the entire stent 1 is resheathed in the catheter 50. Thus, the stent 1 can be retrieved from the body together with the catheter 50.

### (Modification)

The present disclosure is not limited to the above-described embodiments and various modifications and changes can be made, and these modifications and changes are also included in the scope of the present disclosure.

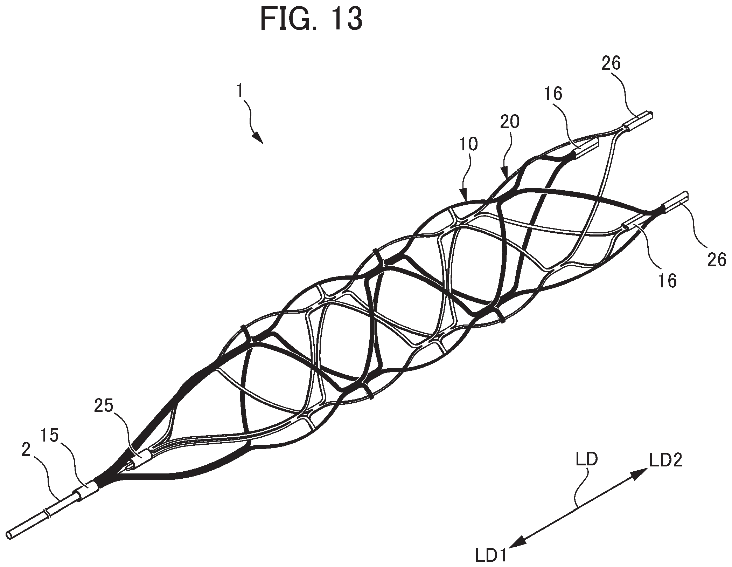

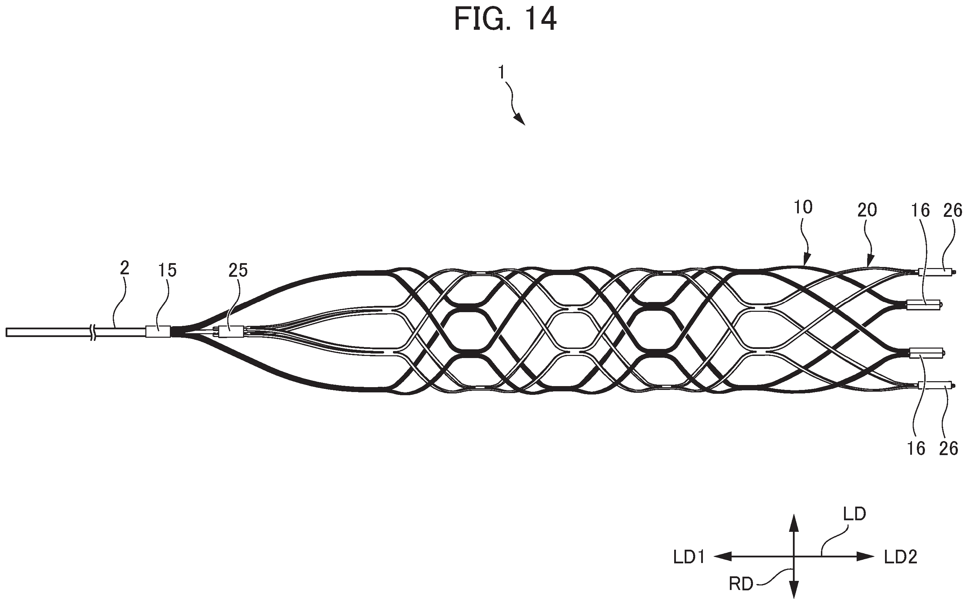

(1) In each embodiment, an example has been described in which, in the distal side LD2 of the stent 1, the end portions of the first stent body 10 and the second stent body 20 are gradually narrowed toward a side of the distal end shaft 3, and are connected to the distal end shaft 3. The present disclosure is not limited thereto, and for example, it may be configured such that the distal side LD2 end portion of the stent 1 is opened. FIG. 13 is a perspective view of the stent 1 in which a distal side LD2 end portion is opened. FIG. 14 is a side view of the stent 1 in which the distal side LD2 end portion is opened. In the stent 1 of each embodiment described above, it may be configured such that the distal side LD2 end portion of the stent 1 is opened as shown in FIGS. 13 and 14.

In the stent 1 of each embodiment described above, an example has been shown in which six cells are arrayed along the circumferential direction OD. The present disclosure is not limited thereto, and for example, the number of cells arrayed along the circumferential direction OD may be reduced as shown in FIGS. 13 and 14. In FIGS. 13 and 14, an example is shown in which two cells are arrayed along the circumferential direction OD.

In the stent 1 of each embodiment described above, the marker is not particularly referred to, but as shown in FIGS. 13 and 14, a marker can be provided as necessary. For example, in the stent 1 shown in FIGS. 13 and 14, markers 15, 16, 25, and 26 are provided. The markers 15, 16, 25, and 26 each are a member serving as a mark for checking the position of the stent 1 in the tubular organ such as a blood vessel, and are formed of a radiopaque material.

The marker 15 is formed in a circular tube shape, and is bonded and fixed to a joining portion of the proximal side LD1 end portion of the first stent body 10. The marker 25 is formed in a circular tube shape, and is bonded and fixed to a joining portion of the proximal side LD1 end portion of the second stent body 20. The markers 15 and 25 are arranged to be shifted in the LD direction. Since the markers 15 and 25 are arranged to be shifted in the LD direction, the position of the proximal side LD1 end portion of each of the first stent body 10 and the second stent body 20 can be correctly checked.

The marker 16 is formed in a substantially square pipe shape, and is crimp-fixed to the distal side LD2 end portion of the first stent body 10. The marker 26 is formed in a substantially square pipe shape, and is crimp-fixed to the distal side LD2 end portion of the second stent body 20. The marker 26 is arranged closer on the distal side LD2 than the marker 16, and in this manner, the position of the distal side LD2 end portion of each of the first stent body 10 and the second stent body 20 can be correctly checked. Note that a form of each marker is not limited to the above-described configuration, and conventionally known markers of various forms are available.

(2) In each embodiment, an example has been described in which the stent has a double-layer structure including the first stent body 10 and the second stent body 20. The present disclosure is not limited thereto, and for example, still another stent body may be provided outside the first stent body 10 and/or inside the second stent body 20. In this case, the relationship among another stent body, the first stent body, and the second stent body may be similar to the relationship between the first stent body and the second stent body in the above-described embodiments.

Note that each embodiment and modification may be combined as necessary, but detailed description thereof will be omitted. The present disclosure is not limited to each embodiment described above.

### EXPLANATION OF REFERENCE NUMERALS

1 Stent
2 Push wire
3 Distal end shaft
10 First stent body
11 First strut
12 First cell
13 Opening portion
14 Intersecting point
20 Second stent body
21 Second strut
22 Second cell
23 Opening portion
24 Intersecting point
t Strut thickness
t1 First strut thickness
t2 Second strut thickness
w Strut width
w1 First strut width
w2 Second strut width

## Claims

1. A stent, comprising:
a first stent body in which a plurality of first cells surrounded by a plurality of first struts are arrayed in a circumferential direction and a central axis direction;
a second stent body in which a plurality of second cells surrounded by a plurality of second struts are arrayed in the circumferential direction and the central axis direction and at least a region that expands a narrowed site of a living body lumen is inserted into the first stent body, wherein
at least one group of a first group of the first struts and a second group of the second struts has a portion in which a strut width w in a direction perpendicular to an extension direction of the first struts or the second struts is wider than a strut thickness t in a direction perpendicular to a central axis.

2. The stent according to claim 1, wherein
the strut width w and the strut thickness t satisfy 0.2 ≤ t/w.

3. The stent according to claim 1 or 2, wherein
the strut width w satisfies 30 µm ≤ w ≤ 250 µm.

4. The stent according to claim 3, wherein
the strut thickness t satisfies 20 µm ≤ t ≤ 150 µm.

5. The stent according to claim 1 or 2, wherein
t1 > t2 is satisfied when the strut thickness t is defined as t1 for the first struts or t2 for the second struts.

6. The stent according to claim 1 or 2, wherein
w1 > w2 is satisfied when the strut width w of the portion is defined as w1 for the first struts or w2 for the second struts.

7. The stent according to claim 1 or 2, wherein
the stent is used for applications in which the stent is temporarily placed in the living body lumen and subsequently retrieved from a body.
